# EUROPÄISCHE PATENTSCHRIFT

(11) **EP 1 680 149 B1**
(45) Veröffentlichungstag und Bekanntmachung des Hinweises auf die Patenterteilung: **02.05.2007**
(21) Anmeldenummer: 04797574.3
(22) Anmeldetag: 03.11.2004
(51) Int. Cl.: A61L 27/28, A61L 31/08

(54) **VERFAHREN ZUR BESCHICHTUNG VON IMPLANTATEN MITTELS EINES DRUCKVERFAHRENS**
METHOD FOR COATING IMPLANTS BY WAY OF A PRINTING METHOD
PROCEDE DE REVETEMENT D'IMPLANTS PAR UN PROCEDE D'IMPRESSION

(30) Priorität: 03.11.2003 DE 10351150
(43) Veröffentlichungstag der Anmeldung: 19.07.2006
(73) Patentinhaber: CINVENTION AG, 65203 Wiesbaden (DE)
(72) Erfinder: KUNSTMANN, Jürgen, 65812 Bad Soden (DE); MAYER, Bernhard, 55130 Mainz (DE); RATHENOW, Jörg, 65203 Wiesbaden (DE); ASGARI, Sohéil, 65193 Wiesbaden (DE)
(74) Vertreter: Hansen, Norbert
(86) Internationale Anmeldenummer: PCT/EP2004/012442
(87) Internationale Veröffentlichungsnummer: WO 2005/042045

(56) Entgegenhaltungen:
- WO-A-01/49337
- US-A1- 2003 003 220
- US-B1- 6 555 157

## Beschreibung

Die vorliegende Erfindung betrifft ein Verfahren und eine Vorrichtung zum Auftragen einer definierten Menge eines Beschichtungsmaterials auf die Oberfläche eines Implantats mittels eines Druckverfahrens, insbesondere unter Verwendung einer Druckwalze. Die Erfindung betrifft ferner die Verwendung eines Druckverfahrens, insbesondere einer Druckwalze zum Auftragen einer definierten Menge eines Beschichtungsmaterials auf die Oberfläche eines zu beschichtenden Implantats, sowie entsprechend hergestellte beschichtete Implantate.

Um körpereigene Abwehrreaktionen gegen Fremdimplantate zu verringern oder möglichst zu vermeiden werden in der Medizin zunehmend beschichtete Implantate verwendet, um die biologische Verträglichkeit der verwendeten Implantatmaterialien zu erhöhen, eine bessere Integration in das umliegende Gewebe zu ermöglichen und das körperfremde Material des Implantats von dem Immunsystem zu "tarnen". Auch werden zunehmend mit pharmakologisch wirksamen Stoffen beschichtete oder imprägnierte Implantate verwendet, welche eine gezielte Freisetzung des Wirkstoffs lokal am Einsatzort des Implantats ermöglichen.

Zur Beschichtung von Implantaten sind im Stand der Technik eine Vielzahl von Verfahren bekannt. Übliche Methoden zum Aufbringen von Beschichtungen auf Implantaten sind beispielsweise Aufstreichen, Lackieren, sowie insbesondere Tauchverfahren und dergleichen.

Die Beschichtung komplex geformter medizinischer Implantate, beispielsweise Koronarstents, Gelenksprothesen, sowie besonders auch chirurgischen Implantate erfordert zunehmend eine hohe Auftragungsgenauigkeit im Hinblick auf die exakte Bestimmung der aufzutragenden Menge der Beschichtung bzw. des Beschichtungsmaterials, insbesondere wenn medizinische Wirkstoffe aufgebracht werden, als auch im Hinblick auf die Qualität und Dauerhaftigkeit der Beschichtung.

Insbesondere zur Beschichtung mit pharmakologischen Wirkstoffen werden bei medizinischen Implantaten oftmals Tauch- oder Tauchimprägnierungsverfahren eingesetzt. Diese Verfahren haben den Nachteil, dass die exakte Menge des aufgenommenen pharmakologischen Wirkstoffs von der Absorptions- oder Adsorptionscharakteristik und der gewählten Beschichtungsbedingungen in hohem Maße abhängt. Hierdurch wird die exakte Bestimmung der tatsächlich aufgetragenen Menge des pharmakologischen Wirkstoffs erschwert, bzw. sie unterliegt verfahrensbedingten Schwankungen. So gibt es üblicherweise Abweichungen zwischen der theoretischen und der praktischen Aufnahmekapazität poröser Implantatoberflächen für jeden einzelnen aufzutragenden Wirkstoff, die teilweise erheblich sein können.

Aus der DE 198 49 467 ist es bekannt, Trägerpolymer-beschichtete Stents mit Cyclodextrinen zu derivatisieren, in welche dann pharmakologische Wirkstoffe eingelagert werden können. Hierbei bestimmt die Menge der oberflächlich aufgebrachten Cyclodextrine die aufnehmbare Menge des Wirkstoffs in relativ gut reproduzierbarer Weise. Dadurch lässt sich die maximal aufzunehmende Dosis des Wirkstoffs in der Beschichtung bestimmen.

Nachteil des Verfahrens der DE 198 49 467 ist jedoch, dass die Oberfläche der Stents mit einem Trägerpolymer beschichtet sein müssen, welches in der Lage ist Cyclodextrine zu binden. Darüber hinaus sind bei diesem Verfahren nach Herstellung der Cyclodextrin-derivatisierten Stentoberfläche Messungen erforderlich, welche die exakte Aufnahmekapazität des Cyclodextrinanteils für pharmakologische Wirkstoffe ermitteln. Bei der tatsächlichen Einbringung von Wirkstoffen in die Cyclodextrine kommt es wie bei anderen absorptiven Systemen auch hier zu Differenzen zwischen theoretischer und praktischer Aufnahmekapazität in Abhängigkeit der verwendeten Wirkstoffe.

Angesichts der Ungenauigkeiten der Dosierung von Wirkstoffen bei der Beschichtung medizinischer Implantate nach bekannten Verfahren besteht ein Bedarf nach einfachen und vielfältig verwendbaren Beschichtungsverfahren, welche eine exakte Dosierung von Wirkstoffen bei der Beschichtung von Formkörpern, insbesondere von medizinischen Implantaten ermöglicht.

Eine Aufgabe der vorliegenden Erfindung ist es daher ein Verfahren zum Auftragen eines Beschichtungsmaterials auf die Oberfläche eines beliebigen Implantats zur Verfügung zu stellen, welches eine exakte Kontrolle über die aufgebrachte Menge des Beschichtungsmaterials ermöglicht.

Eine weitere Aufgabe der Erfindung ist es ein entsprechendes Auftragungsverfahren bereitzustellen, mit dem Implantate ein- oder mehrfach, d. h. mit einer oder mehreren Schichten eines oder verschiedener Beschichtungsmaterialien in exakter und reproduzierbarer Weise beschichtet werden können.

Eine weitere Aufgabe der vorliegenden Erfindung besteht in der Bereitstellung einer Vorrichtung zur Ausführung des erfindungsgemäßen Auftragungsverfahren.

Die erfindungsgemäße Lösung der oben genannten Aufgaben ergibt sich aus den unabhängigen Verfahrens- und Vorrichtungsansprüchen.

Bevorzugte Ausführungsformen ergeben sich durch Kombination mit den Merkmalen der abhängigen Ansprüche.

Die erfindungsgemäße Lösung der verfahrensbezogenen Aufgabe der vorliegenden Erfindung besteht in einem Verfahren zum Auftragen einer definierten Menge eines Beschichtungsmaterials auf die Oberfläche eines zu beschichtenden Implantats mittels eines Druckverfahrens, umfassend die Schritte:
- Beladen der in der Mantelfläche einer Druckwalze ausgebildeten Vertiefungen mit einer definierten Menge des Beschichtungsmaterials;
- Anordnen der Druckwalze bei dem zu beschichtenden Implantat in der Weise, dass die der Oberflächenbeschaffenheit des zu beschichtenden Implantats immanenten Adsorptions- bzw. Adhäsionskräfte ausreichen, um das in den Vertiefungen der Mantelfläche der Druckwalze befindliche Beschichtungsmaterial anziehen zu können;
- Auftragen des in den Vertiefungen der Mantelfläche der Druckwalze befindliche Beschichtungsmaterials durch Entlangbewegen der Mantelfläche der Druckwalze und der Oberfläche des zu beschichtenden Implantats.

Die vorrichtungsbezogene erfindungsgemäße Lösung obengenannter Aufgaben liegt in einer Vorrichtung zum Auftragen einer definierten Menge eines Beschichtungsmaterials auf die Oberfläche eines zu beschichtenden Implantats mit einer Druckwalze, in deren Mantelfläche eine Vielzahl an Vertiefungen ausgebildet sind, um eine definierte Menge an Beschichtungsmaterial aufnehmen zu können, wobei die Druckwalze stets bei dem zu beschichtenden Implantat so angeordnet ist, dass die der Oberflächenbeschaffenheit des zu beschichtenden Implantats immanenten Saug- oder Adsorptionskräfte ausreichen, um das in den Vertiefungen der Mantelfläche der Druckwalze befindliche Beschichtungsmaterial anziehen zu können, um durch Entlangbewegen der Mantelfläche der Druckwalze und der Oberfläche des zu beschichtenden Körpers das in den Vertiefungen der Mantelfläche der Druckwalze befindliche Beschichtungsmaterial auf die Oberfläche des zu beschichtenden Implantats aufzutragen. Vorzugsweise erfolgt das Entlangbewegen der Mantelfläche der Druckwalze und der Oberfläche des zu beschichtenden Körpers im wesentlichen schlupffrei.

Erfindungsgemäß wurde gefunden, dass sich Druckverfahren in besonderer Weise dazu eignen, Beschichtungsmaterialien auf die Oberfläche eines zu beschichtenden Implantats in definierter und bezüglich der Menge exakt dosierter Weise aufzubringen.

Vorzugsweise werden hierzu Druckwalzen mit definierter Oberflächenstruktur verwendet, die in der Mantelfläche der Druckwalze Vertiefungen aufweisen, welche eine exakte Bestimmung des Volumens des Beschichtungsmaterials pro Flächeneinheit der Druckwalze ermöglichen.

Mit dem Begriff Druckwalze sind in der vorliegenden Erfindung alle Druckwalzen gemeint, deren Mantelfläche eine Vielzahl von Vertiefungen definierter Geometrie und Anordnung enthält. Die Vertiefungen in der Mantelfläche der Druckwalze können dabei nahezu beliebige dreidimensionale geometrische Formen aufweisen, wie beispielsweise Näpfchen, Rillenstrukturen, Spitzpyramiden, Stumpfpyramiden, Raster, Halbkugelraster, zylinderförmige Vertiefungen und dergleichen.

Die in der Mantelfläche der Druckwalze ausgebildeten Vertiefungen ermöglichen es anhand ihrer bekannten Dimensionen das Volumen eines Beschichtungsmaterials das auf der Druckwalze aufgebracht wird, bezogen auf die Flächeneinheit der Mantelfläche der Druckwalze, eindeutig und mit hoher Genauigkeit zu bestimmen. Das Vertiefungsvolumen pro Flächeneinheit in der Mantelfläche der Druckwalze ergibt damit ein exaktes Maß für die maximale Dosis des Beschichtungsmaterials, das beim Auftragen des in den Vertiefungen der Mantelfläche der Druckwalze befindlichen Beschichtungsmaterials auf die Oberfläche des zu beschichtenden Implantats abgegeben werden kann.

Auf diese Weise kann aus dem Vertiefungsvolumen der Druckwalze pro Flächeneinheit die maximale Beschichtungsmaterialmenge welche auf die Oberfläche des zu bestimmenden Implantats beim Entlangbewegen des Implantats an der Mantelfläche der Druckwalze oder beim Entlangbewegen der Druckwalze an der Oberfläche des zu beschichtenden Implantats übertragen wird, exakt bestimmt werden. Durch mehrfaches Entlangbewegen der Mantelfläche der Druckwalze und der Oberfläche des zu beschichtenden Implantats kann die Dosis des Beschichtungsmaterials beliebig vervielfacht werden.

Erfindungsgemäß verwendbare Druckwalzen können ausgewählt werden aus Gravurwalzen, Rasterwalzen, Tiefdruckwalzen, Keramikwalzen, Keramikrasterwalzen, keramikbeschichtete Rasterwalzen, Flexodruckwalzen, Prägewalzen, Kalanderwalzen, sowie sonstiger Druckwalzen, deren Mantelflächen Vertiefungen zur Aufnahme von Beschichtungsmaterial aufweisen, besonders bevorzugt Rasterwalzen.

Daneben besteht nach einem weiteren Aspekt der vorliegenden Erfindung auch die Möglichkeit, Druckwalzen ohne Vertiefungen, d.h. mit glatter Oberflächenstruktur zu verwenden, auf welche das Beschichtungsmaterial mittels geeigneter Verfahren mit einer definierten Schichtdicke aufgebracht wird. Derartige Verfahren zur Beladung von Druckwalzen mit definierten Schichtdicken von Beschichtungsmaterial sind im Stand der Technik bekannt und dem Druckfachmann geläufig. Alle bisher und im folgenden im Zusammenhang mit Vertiefungen enthaltenden Druckwalzen beschriebenen Merkmale gelten prinzipiell, ggf. entsprechend angepasst, auch für vertiefungsfreie Druckwalzen und damit durchgeführte Auftragungsverfahren.

Die Beschichtungsdicke auf der vertiefungsfreien Druckwalze lässt sich hierbei mit dem Fachmann bekannten Verfahren einstellen, beispielsweise unter Verwendung von Präzisionssprühtechnologie oder Ultraschallzerstäubungsverfahren für sehr feinverteilte und homogene Sprühbilder.

Nach dem erfindungsgemäßen Auftragungsverfahren werden zunächst die in der Mantelfläche der Druckwalze ausgebildeten Vertiefungen bzw. die Mantelfläche der Druckwalze selbst mit einer definierten Menge des Beschichtungsmaterials beladen. Dies kann in Abhängigkeit vom Aggregatszustand des Beschichtungsmaterials auf vielfältige Art und Weise erfolgen, beispielsweise durch teilweises oder vollständiges Eintauchen der Druckwalzenoberfläche in flüssige oder pulverförmige Beschichtungsmaterialien, Aufsprühen flüssiger, gelöster oder pulverförmiger Beschichtungsmaterialien auf die Mantelfläche der Druckwalze und dergleichen. In besonders bevorzugten Ausführungsformen können pulverförmige Beschichtungsmaterialien auch durch elektrostatische Anziehung auf die Mantelfläche und in deren Vertiefungen aufgebracht werden.

Um das Volumen des Beschichtungsmaterials in den Vertiefungen in der Mantelfläche der Druckwalze möglichst exakt einzustellen ist es bevorzugt nach dem Auftragen des Beschichtungsmaterials auf die Druckwalze mögliche Beschichtungsmaterialüberschüsse von der Mantelfläche zu entfernen. Dies kann im einfachsten Fall dadurch geschehen, dass mit einem Messerbalken oder ähnlichen Vorrichtungen die Vertiefungen auf das Niveau der Walzenoberfläche abgerakelt bzw. abgestrichen werden.

Auch ist es möglich durch ein sehr feines Muster der Vertiefungen und deren Rasterung eine genaue, reproduzierbare Dosierung der Auftragssubstanz zu gewährleisten. In bevorzugten Ausführungsformen des erfindungsgemäßen Verfahrens ist die obere Oberfläche der Vertiefungen kleiner als die zu beschichtende Fläche des Implantats. Das Verhältnis der oberen Oberfläche der Vertiefungen in der Druckwalze zur Oberfläche des zu beschichtenden Implantats beträgt vorzugsweise 1:10, besonders bevorzugt 1:100, insbesondere bevorzugt 1:1000, 1:5000, 1:10000 oder mehr.

Besonders bevorzugt ist die Verwendung von Rasterwalzen, insbesondere Keramikrasterwalzen oder keramikbeschichteter Rasterwalzen, sowie auch die Verwendung von Gravurwalzen aus Metall, insbesondere Edelstahl. Besonders bevorzugte Walzen sind ferner Rasterwalzen aus Stahl, gegebenenfalls verchromt, bzw. Edelstahl. In bevorzugten Ausführungsformen werden insbesondere Edelstahlrasterwalzen bzw. verchromte Stahlrasterwalzen mit einer Rasterung von 120, 240 und bis zu 300 verwendet, d.h. mit 120 x 120, 240 x 240 oder 300 x 300 Vertiefungen pro cm³ der Druckwalzenmantelfläche. Das Volumen der Vertiefungen beträgt üblicherweise von 1x10⁻⁶ mm³ bis 1x10⁻⁴ mm³, kann jedoch vom Fachmann je nach gewünschtem Anwendungszweck auch größer oder kleiner gewählt werden, indem z.B. eine höhere Rasterung oder tiefere bzw. größere Vertiefungen eingesetzt werden. Das Vertiefungsvolumen einer erfindungsgemäß verwendbaren Edelstahlrasterwalze beträgt vorzugsweise bei einer 240er Rasterung etwa 2x10⁻⁵ mm³.

Bei Keramikrasterwalzen oder keramikbeschichteten Rasterwalzen sind bevorzugte Rasterungen bei 120, 450 bis zu 700, bei Vertiefungsvolumina von 1x10⁻⁷ mm³ bis 1x10⁻⁴ mm³, vorzugsweise 5,7x10⁻⁶ mm³, wobei auch hier vom Fachmann je nach gewünschtem Anwendungszweck auch größere oder kleinere Vertiefungsvolumina gewählt werden können, indem z.B. eine höhere Rasterung oder tiefere bzw. größere Vertiefungen eingesetzt werden.

Gemäß einer bevorzugten Ausführungsform der Erfindung geschieht das Beladen der in der Mantelfläche der Druckwalze ausgebildeten Vertiefungen mit einem Beschichtungsmaterial über eine rotierende Schöpfwalze, wobei sich während der Rotation zumindest ein Zylindersegment der Schöpfwalze ständig in einem Beschichtungsmaterialbad befindet, wodurch die Schöpfwalze umfangseitig mit Beschichtungsmaterial benetzt wird, und die Schöpfwalze das so aufgenommene Beschichtungsmaterial anschließend auf die Druckwalze überträgt. Bevorzugt ist es, wenn die Schöpfwalze hierbei die Druckwalze berührt, so dass überschüssiges Beschichtungsmaterial im Wesentlichen von der Oberfläche der Druckwalze abgequetscht wird. Gegebenenfalls kann ersatzweise oder zusätzlich die Oberfläche der Schöpfwalze auch abgestrichen werden, beispielsweise mit einem Messerbalken oder dergleichen.

Die mit Beschichtungsmaterial in definierter Menge beladene Druckwalze wird zu dem zu beschichtenden Implantat so angeordnet, dass die der Oberflächenbeschaffenheit des zu beschichtenden Implantats immanenten Adsorptions- bzw. Adhäsionskräfte ausreichen, um das in den Vertiefungen der Mantelfläche der Druckwalze befindliche Beschichtungsmaterial anziehen zu können, d. h. aus den Vertiefungen der Druckwalzenmantelfläche entfernen und auf der Oberfläche des zu beschichtenden Implantats anzuhaften bzw. zu fixieren oder im Porensystem einer porösen Implantatoberfläche aufzusaugen.

Gemäß einer Ausführungsform der Erfindung erfolgt die Positionierung der beladenen Druckwalze zu dem zu beschichtenden Implantat in der Weise, dass zwischen Implantat und Druckwalze ein direkter Kontakt erfolgt.

Gemäß einer alternativen Ausführungsform der vorliegenden Erfindung, welche gegenwärtig bevorzugt ist, erfolgt die Positionierung der beladenen Druckwalze zu dem zu beschichtenden Implantat ohne direkten Kontakt. Hierbei kommen sich Druckwalze und zu beschichtender Implantat ausreichend nahe, dass die in den Vertiefungen der Mantelfläche der Druckwalze befindlichen Beschichtungsmaterialvolumina von der Druckwalze auf den zu beschichtenden Implantat übergehen können, vorzugsweise im Wesentlichen vollständig. In Abhängigkeit von den spezifischen Eigenschaften des jeweils verwendeten Beschichtungsmaterials und der Oberflächenbeschaffenheit des Implantats wird der Fachmann die beste Geometrie für ein kontaktfreies Auftragsverfahren im Wege von Routineversuchen einfach ermitteln können.

Bei flüssigen Beschichtungsmaterialien sind beispielsweise Abstände zwischen Druckwalze und Implantat von 1 µm bis 10 mm möglich, vorzugsweise etwa 100 µm.

Zum Auftragen des in den Vertiefungen der Mantelfläche der Druckwalze befindlichen Beschichtungsmaterials ist es bevorzugt, dass die Bewegung zwischen Mantelfläche der Druckwalze und der Oberfläche des zu beschichtenden Implantats schlupffrei erfolgt. Das erfindungsgemäße Verfahren kann so durchgeführt werden, dass die Oberfläche des zu beschichtenden Implantats schlupffrei an der Mantelfläche der Druckwalze entlang bewegt wird, oder dass die Mantelfläche der Druckwalze schlupffrei an der Oberfläche des zu beschichtenden Implantats entlang bewegt wird. Auch eine vorzugsweise schlupffreie Gegeneinanderbewegung zwischen Mantelfläche der Druckwalze und Oberfläche des zu beschichtenden Implantats ist erfindungsgemäß möglich und in bestimmten Ausfühningsformen des erfindungsgemäßen Verfahrens besonders bevorzugt.

Sofern das Entlangbewegen der Mantelfläche der Druckwalze und der Oberfläche des zu beschichtenden Körpers nicht schlupffrei erfolgt, müssen die Bedingungen des Übergangs von Beschichtungsmaterial von der Druckwalze auf das Implantat so eingestellt werden, dass eine reproduzierbare Menge an Beschichtungsmaterial pro Bewegungsablauf übertragen wird. Hierzu sind bei Flüssigsystemen die hydrodynamischen Bedingungen entsprechend anzupassen.

In einer besonders bevorzugten Ausführungsform der Erfindung ist das zu beschichtende Implantat selbst von zylindrischer Form, so dass das vorzugsweise schlupffreie Entlangbewegen der Mantelfläche der Druckwalze und der Oberfläche des zu beschichtenden Implantats dadurch erfolgt, dass die Druckwalze und das zu beschichtende Implantat um zwei im Wesentlichen parallel zueinander liegende Achsen gegensinnig gedreht werden.

Bei nichtzylindrischen Geometrien des zu beschichtenden Implantats kann das vorzugsweise schlupffreie Entlangbewegen der Mantelfläche der Druckwalze und der Oberfläche des zu beschichtenden Implantats so erfolgen, dass die Achse der Druckwalze äquidistant zur Oberfläche des zu beschichtenden Implantats entlang bewegt wird. Auf diese Weise erfolgt quasi eine Abtastung der Oberfläche des zu beschichtenden Implantats mit der beladenen Druckwalze.

Das nach dem erfindungsgemäßen Verfahren zu beschichtende Implantat kann prinzipiell beliebige Formen annehmen, sofern das Verfahren vorrichtungstechnisch daran angepasst wird. Hierzu stehen dem Fachmann eine Vielzahl von möglichen Anordnungen der Druckwalze und des Beladungssystems für die Vertiefungen in der Mantelfläche der Druckwalze zur Verfügung, die er je nach Bedarf auswählen wird.

Der Begriff "Implantat" wird im Rahmen der vorliegenden Beschreibung so verwendet, dass er allgemein medizinische, diagnostische oder therapeutische Implantate wie beispielsweise Gefäßendoprothesen, intraluminale Endoprothesen, Stents, Koronarstents, periphere Stents, chirurgische bzw. orthopädische Implantate für temporäre Zwecke wie chirurgische Schrauben; Platten, Nägel und sonstige Befestigungsmittel, permanente chirurgische oder orthopädische Implantate wie Knochen- oder Gelenkprothesen, beispielsweise künstliche Hüft- oder Kniegelenke, Gelenkpfanneneinsätze, Schrauben, Platten, Nägel, implantierbare orthopädische Fixierungshilfsmittel, Wirbelkörperersatzmittel, sowie Kunstherzen und Teile davon, künstliche Herzklappen, Herzschrittmachergehäuse, Elektroden, perkutane, subkutane und/oder intramuskulär einsetzbare Implantate, Wirkstoffdepots und Mikrochips, und dergleichen umfasst, die dazu gedacht sind in den menschlichen oder tierischen Körper eingesetzt zu werden bzw. zur Anwendung am oder im menschlichen oder tierischen Körper gedacht sind.

Erfindungsgemäß bevorzugt handelt es sich bei dem zu beschichtenden Implantat um medizinische, diagnostische oder therapeutische Implantate, wie Gefäßendoprothesen, Stents, Koronarstents, peripheren Stents, orthopädischen Implantate, Knochen- oder Gelenkprothesen, Kunstherzen, künstliche Herzklappen, Herzschrittmacherelektroden, Subkutane, Perkutane und/oder intramuskuläre Implantate, chirurgische Nägel, Schrauben, Befestigungsmittel, Pins und dergleichen.

Jedoch kann prinzipiell jeder beliebige Formkörper mit dem erfindungsgemäßen Verfahren/der Vorrichtung beschichtet werden, wobei sich das erfindungsgemäße Verfahren insbesondere dadurch auszeichnet, dass die Menge des aufgetragenen Beschichtungsmaterials mit großer Exaktheit bestimmt und vorgegeben werden kann.

Gemäß einer besonders bevorzugten Ausführungsform der Erfindung ist das zu beschichtende Implantat ein Stent, insbesondere bevorzugt von im Allgemeinen zylindrischer Form, besonders bevorzugt ein Kohlenstoff-beschichteter Stent, wie beispielsweise in der DE 103 33 098 beschrieben und nach dem dortigen Verfahren hergestellt.

Die mit dem Verfahren der vorliegenden Erfindung reproduzierbar beschichtbaren Implantate können aus nahezu beliebigen Materialien bestehen, insbesondere aus allen Materialien, aus denen Implantate hergestellt werden.

Beispiele hierfür sind amorpher und/oder (teil-)kristalliner Kohlenstoff, Vollkarbonmaterial, poröser Kohlenstoff, Graphit, Kohlenstoffverbundmaterialien, Kohlefasern, Kunststoff, Polymermaterial, Kunststofffasern, Keramiken wie z. B. Zeolithe, Silikate, Aluminiumoxide, Aluminosilikate, Siliziumkarbid, Siliziumnitrid; Metallkarbide, Metalloxide, Metallnitride, Metallcarbonitride, Metalloxycarbide, Metalloxynitride und Metalloxycarbonitride der Übergangsmetalle wie Titan, Zirkonium, Hafnium, Vanadium, Niob, Tantal, Chrom, Molybdän, Wolfram, Mangan, Rhenium, Eisen, Kobalt, Nickel; Metalle und Metalllegierungen, insbesondere der Edehnetalle Gold, Silber, Ruthenium, Rhodium, Palladium, Osmium, Iridium, Platin; Metalle und Metalllegierungen von Titan, Zirkon, Hafnium, Vanadin, Niob, Tantal, Chrom, Molybdän, Wolfram, Mangan, Rhenium, Eisen, Kobalt, Nickel, Kupfer; Stahl, insbesondere rostfreier Stahl, Formgedächtnislegierungen wie Nitinol, Nickel-Titanlegierung, Glas, Stein, Glasfasern, Mineralien, natürliche oder synthetische Knochensubstanz, Knochenimitate auf Basis von Erdalkalimetallkarbonaten wie Kalziumkarbonat, Magnesiumkarbonat, Strontiumkarbonat, sowie beliebige Kombinationen der genannten Materialien.

Das zu beschichtende Implantat kann in Abhängigkeit von dem aufzutragenden Beschichtungsmaterial aus beliebigen Stoffen bestehen, sofern das Material in der Lage ist das Beschichtungsmaterial aufzunehmen und/oder zu binden bzw. oberflächlich zu fixieren.

Bevorzugte Materialien aus dem Bereich der medizinischen, diagnostischen oder therapeutischen Implantate, welche nach dem erfindungsgemäßen Verfahren beschichtet werden können, sind beispielsweise Kohlenstoff, Kohlefasern, Volllcarbonmaterial, Kohlenstoffverbundmaterial, Kohlefaser, Kunststoff, Polymermaterial, Kunststofffasern, Keramik, Glas oder Glasfasern, Metalle wie rostfreier Stahl, Tital, Tantal, Platin; Legierungen wie Nitinol, Nickel-Titan Legierung; Knochen, Stein, Mineral oder Kombinationen dieser Materialien, aus welchen das zu beschichtende Implantat besteht. Gegebenenfalls können die zu beschichtenden Implantate aus den oben genannten Materialien auch bereits mit einer oder mehreren Schichten aus einem oder mehreren der genannten Materialien beschichtet sein.

Das Beschichtungsmaterial zur Verwendung im erfindungsgemäßen Verfahren kann eine Lösung, Suspension oder Emulsion eines oder mehrerer Wirkstoffe oder Wirkstoffvorstufen in einem geeigneten Trägermedium sein, ein unverdünnter flüssiger Wirkstoff oder auch einer oder mehrerer Wirkstoffe und Wirkstoffvorstufen in Pulverform.

Unter dem Begriff "Wirkstoffe" werden erfindungsgemäß pharmakologisch wirksame Stoffe wie Arzneimittel, Medikamente, Pharmaka, aber auch Mikroorganismen, lebendes organisches Zellmaterial, Enzyme sowie auch biologisch verträgliche anorganische oder organische Stoffe verstanden. Mit dem Begriff "Wirkstoffvorstufen" werden Stoffe oder Stoffgemische bezeichnet, welche nach der Auftragung auf ein zu beschichtendes Implantat mittels thermischer, mechanischer oder chemischer bzw. biologischer Prozesse in Wirkstoffe der oben genannten Art umgewandelt werden.

Die in den Beschichtungsmaterialien des erfindungsgemäßen Verfahrens verwendbaren Wirkstoffe oder Wirkstoffvorstufen organischer Art können biologisch abbaubare bzw. resorbierbare Polymere wie Kollagen, Albumin, Gelatin, Hyaluronsäure, Stärke, Cellulosen wie Methylcellulose, Hydroxypropylcellulose, Hydroxypropylmethylcellulose, Carboxymethylcellulose-Phtalat; Kasein, Dextrane, Polysaccharide, Fibrinogen, Poly(D,L-Lactide), Poly(D,L-Lactide-Co-Glycolide), Poly(Glycolide), Poly(Hydroxybutylate), Poly(Alkylcarbonate), Poly(Orthoester), Polyester, Poly(Hydroxyvalerinsäure), Polydioxanone, Poly(Ethylenterephtalate), Poly(malatsäure), Poly(Tartronsäure), Polyanhydride, Polyphosphazene, Poly(Aminosäuren), und deren Co-Polymere oder nicht-biologisch abbaubare bzw. resorbierbare Polymere sein. Bevorzugt sind insbesondere anionische, kationischen oder amphotere Beschichtungen, wie z.B. Alginat, Carrageenan, Carboxymethylcellulose; Chitosan, Poly-L-Lysine; und/oder Phoshporylcholin.

Als Beschichtungsmaterial gemäß der vorliegenden Erfindung verwendbare Wirkstoffe oder Wirkstoffvorstufen können auch Marker, Kontrastmittel oder dergleichen sein, die zur Lokalisierung beschichteter Implantate im Körper verwendet werden können, beispielsweise therapeutische oder diagnostische Mengen an radioaktiven Strahlern und dergleichen.

In bestimmten Ausführungsformen, insbesondere bei subkutanen/intramuskulären Wirkstoffdepots oder Stents, kann die Wirkstoffbeladung auch temporär sein, d. h. der Wirkstoff wird nach Implantierung des Implantats freigesetzt, oder aber der Wirkstoff wird in oder auf dem Implantat dauerhaft immobilisiert. Auf diese Weise können wirkstoffhaltige medizinische Implantate mit statischen, dynamischen oder kombiniert statischen und dynamischen Wirkstoffbeladungen erzeugt werden. So ergeben sich multifunktionale Beschichtungen auf den erfindungsgemäß beschichteten Implantaten.

Bei statischer Beladung mit Wirkstoffen werden Wirkstoffe im Wesentlichen permanent auf dem Implantat immobilisiert. Hierfür verwendbare Wirkstoffe sind bioverträgliche anorganische Substanzen, z.B. Hydroxylapatit (HAP), Fluorapatit, Trikalziumphosphat (TCP), Zink; und/oder organische Substanzen wie Peptide, Proteine, Kohlenhydrate wie Mono-, Oligo- und Polysaccharide, Lipide, Phospholipide, Steroide, Lipoproteine, Glykoproteine, Glykolipide, Proteoglykane, DNA, RNA, Signalpeptide oder Antikörper bzw. Antikörperfragmente, bioresorbierbare Polymere, z.B. Polylactonsäure, Chitosan, sowie pharmakologisch wirksame Stoffe oder Stoffgemische, und Kombinationen dieser.

Bei dynamischen Wirkstoffbeladungen ist die Freisetzung der aufgebrachten Wirkstoffe nach dem Einsetzen des Implantats im Körper vorgesehen. Auf diese Weise können die beschichteten Implantate zu therapeutischen Zwecken eingesetzt werden, wobei die auf das Implantat aufgebrachten Wirkstoffe lokal am Einsatzort des Implantats sukzessive freigesetzt werden. In dynamischen Wirkstoffbeladungen für die Freisetzung von Wirkstoffen verwendbare Wirkstoffe sind beispielsweise Hydroxylapatit (HAP), Fluorapatit, Trikalziumphosphat (TCP), Zink; und/oder organische Substanzen wie Peptide, Proteine, Kohlenhydrate wie Mono-, Oligo- und Polysaccharide, Lipide, Phospholipide, Steroide, Lipoproteine, Glykoproteine, Glykolipide, Proteoglykane, DNA, RNA, Signalpeptide oder Antikörper bzw. Antikörperfragmente, bioresorbierbare Polymere, z.B. Polylactonsäure, Chitosan, und dergleichen, sowie pharmakologisch wirksame Stoffe oder Stoffgemische.

Geeignete pharmakologisch wirksame Stoffe oder Stoffgemische zur statischen und/oder dynamischen Beladung von erfindungsgemäß beschichteten Implantaten umfassen Wirkstoffe oder Wirkstoffkombinationen, die ausgewählt sind aus Heparin, synthetische Heparin-Analoga (z.B. Fondaparinux), Hirudin, Antithrombin III, Drotrecogin alpha; Fibrinolytica wie Alteplase, Plasmin, Lysokinasen, Faktor XIIa, Prourokinase, Urokinase, Anistreplase, Streptokinase; Thrombozytenaggregationshemmer wie Acetylsalicylsäure, Ticlopidine, Clopidogrel, Abciximab, Dextrane; Corticosteroide wie Alclometasone, Amcinonide, Augmented Betamethasone, Beclomethasone, Betamethasone, Budesonide, Cortisone, Clobetasol, Clocortolone, Desonide, Desoximetasone, Dexamethasone, Flucinolone, Fluocinonide, Flurandrenolide, Flunisolide, Fluticasone, Halcinonide, Halobetasol, Hydrocortisone, Methylprednisolone, Mometasone, Prednicarbate, Prednisone, Prednisolone, Triamcinolone; sogenannte Non-Steroidal Anti-Inflammatory Drugs wie Diclofenac, Diflunisal, Etodolac, Fenoprofen, Flurbiprofen, Ibuprofen, Indomethacin, Ketoprofen, Ketorolac, Meclofenamate, Mefenamic acid, Meloxicam, Nabumetone, Naproxen, Oxaprozin, Piroxicam, Salsalate, Sulindac, Tolmetin, Celecoxib, Rofecoxib; Zytostatika wie Alkaloide und Podophyllumtoxine wie Vinblastin, Vincristin; Alkylantien wie Nitrosoharnstoffe, Stickstofflost-Analoga; zytotoxische Antibiotika wie Daunorubicin, Doxorubicin und andere Anthrazykline und verwandte Substanzen, Bleomycin, Mitomycin; Antimetabolite wie Folsäure-, Purin- oder Pyrimidin-Analoga; Paclitaxel, Docetaxel, Sirolimus; Platinverbindungen wie Carboplatin, Cisplatin oder Oxaliplatin; Amsacrin, Irinotecan, Imatinib, Topotecan, Interferon-alpha 2a, Interferon-alpha 2b, Hydroxycarbamid, Miltefosin, Pentostatin, Porfimer, Aldesleukin, Bexaroten, Tretinoin; Antiandrogene, und Antiöstrogene; Antiarrythmika, insbesondere Antiarrhythmika der Klasse I wie Antiarrhythmika vom Chinidintyp, z.B. Chinidin, Dysopyramid, Ajmalin, Prajmaliumbitartrat, Detajmiumbitartrat; Antiarrhythmika vom Lidocaintyp, z.B. Lidocain, Mexiletin, Phenytoin, Tocainid; Antiarrhythmika der Klasse I C, z.B. Propafenon, Flecainid(acetat); Antiarrhythmika der Klasse II, Betarezeptorenblocker wie Metoprolol, Esmolol, Propranolol, Metoprolol, Atenolol, Oxprenolol; Antiarrhythmika der Klasse III wie Amiodaron, Sotalol; Antiarrhythmika der Klasse IV wie Diltiazem, Verapamil, Gallopamil; andere Antiarrhythmika wie Adenosin, Orciprenalin, Ipratropiumbromid; Agenzien zur Stimulation der Angiogenese im Myokard wie Vascular Endothelial Growth Factor (VEGF), Basic Fibroblast Growth Factor (bFGF), nicht virale DNA, virale DNA, endotheliale Wachstumsfaktoren: FGF-1, FGF-2, VEGF, TGF; Antikörper, Monoklonale Antikörper, Anticaline; Stammzellen, Endothelial Progenitor Cells (EPC); Digitalisglykoside wie Acetyldigoxin/Metildigoxin, Digitoxin, Digoxin; Herzglykoside wie Ouabain, Proscillaridin; Antihypertonika wie zentral wirksame antiadrenerge Substanzen, z.B. Methyldopa, Imidazolinrezeptoragonisten; Kalciumkanalblocker vom Dihydropyridintyp wie Nifedipin, Nitrendipin; ACE-Hemmer: Quinaprilat, Cilazapril, Moexipril, Trandolapril, Spirapril, Imidapril, Trandolapril; Angiotensin-II-Antagonisten: Candesartancilexetil, Valsartan, Telmisartan, Olmesartanmedoxomil, Eprosartan; peripher wirksame alpha-Rezeptorenblocker wie Prazosin, Urapidil, Doxazosin, Bunazosin, Terazosin, Indoramin; Vasodilatatoren wie Dihydralazin, Diisopropylamindichloracetat, Minoxidil, Nitroprussidnatrium; andere Antihypertonika wie Indapamid, Co-Dergocrinmesilat, Dihydroergotoxinmethansulfonat, Cicletanin, Bosentan, Fludrocortison; Phosphodiesterasehemmer wie Milrinon, Enoximon und Antihypotonika, wie insbesondere adrenerge und dopaminerge Substanzen wie Dobutamin, Epinephrin, Etilefrin, Norfenefrin, Norepinephrin, Oxilofrin, Dopamin, Midodrin, Pholedrin, Ameziniummetil; und partielle Adrenozeptor-Agonisten wie Dihydroergotamin; Fibronectin, Polylysine, Ethylenevinylacetate, inflammatorische Zytokine wie: TGFβ, PDGF, VEGF, bFGF, TNFα, NGF, GM-CSF, IGF-a, IL-1, IL-8, IL-6, Growth Hormone; sowie adhäsive Substanzen wie Cyanacrylate, Beryllium, Silica; und Wachstumsfaktoren (Growth Factor) wie Erythropoetin, Hormonen wie Corticotropine, Gonadotropine, Somatropin, Thyrotrophin, Desmopressin, Terlipressin, Oxytocin, Cetrorelix, Corticorelin, Leuprorelin, Triptorelin, Gonadorelin, Ganirelix, Buserelin, Nafarelin, Goserelin, sowie regulatorische Peptide wie Somatostatin, Octreotid; Bone and Cartilage Stimulating Peptides, sogenannte "bone morphogenic proteins" (BMPs), insbesondere rekombinante BMP's wie z.B. Recombinant human BMP-2 (rhBMP-2)), Bisphosphonate (z.B. Risedronate, Pamidronate, Ibandronate, Zoledronsäure, Clodronsäure, Etidronsäure, Alendronsäure, Tiludronsäure), Fluoride wie Dinatriumfluorophosphat, Natriumfluorid; Calcitonin, Dihydrotachystyrol; Growth Factors und Zytokine wie Epidermal Growth Factor (EGF), Platelet-Derived Growth Factor (PDGF), Fibroblast Growth Factors (FGFs), Transforming Growth Factors-b TGFs-b), Transforming Growth Factor-a (TGF-a), Erythropoietin (Epo), Insulin-Like Growth Factor-I (IGF-I), Insulin-Like Growth Factor-II (IGF-II), Interleukin-1 (IL-1), Interleukin-2 (IL-2), Interleukin-6 (IL-6), Interleukin-8 (IL-8), Tumor Necrosis Factor-a (TNF-a), Tumor Necrosis Factor-b (TNF-b), Interferon-g (INF-g), Colony Stimulating Factors (CSFs); Monocyte chemotactic protein, fibroblast stimulating factor 1, Histamin, Fibrin oder Fibrinogen, Endothelin-1, Angiotensin II, Kollagene, Bromocriptin, Methylsergid, Methotrexat, Kohlenstofftetrachlorid, Thioacetamid, und Ethanol; ferner Silber(ionen), Titandioxid, Antibiotika und Antiinfektiva wie insbesondere β-Laktam-Antibiotika, z.B. β-Lactamase-sensitive Penicilline wie Benzylpenicilline (Penicillin G), Phenoxymethylpenicillin (Penicillin V); β-Lactamaseresistente Penicilline wie Aminopenicilline wie Amoxicillin, Ampicillin, Bacampicillin; Acylaminopenicilline wie Mezlocillin, Piperacillin; Carboxypenicilline, Cephalosporine wie Cefazolin, Cefuroxim, Cefoxitin, Cefotiam, Cefaclor, Cefadroxil, Cefalexin, Loracarbef, Cefixim, Cefuroximaxetil, Ceftibuten, Cefpodoximproxetil, Cefpodoximproxetil; Aztreonam, Ertapenem, Meropenem; β-Lactamase-Inhibitoren wie Sulbactam, Sultamicillintosilat; Tetracycline wie Doxycyclin, Minocyclin, Tetracyclin, Chlortetracyclin, Oxytetracyclin; Aminoglykoside wie Gentamicin, Neomycin, Streptomycin, Tobramycin, Amikacin, Netilmicin, Paromomycin, Framycetin, Spectinomycin; Makrolidantibiotika wie Azithromycin, Clarithromycin, Erythromycin, Roxithromycin, Spiramycin, Josamycin; Lincosamide wie Clindamycin, Lincomycin, Gyrasehemmer wie Fluorochinolone wie Ciprofloxacin, Ofloxacin, Moxifloxacin, Norfloxacin, Gatifloxacin, Enoxacin, Fleroxacin, Levofloxacin; Chinolone wie Pipemidsäure; Sulfonamide, Trimethoprim, Sulfadiazin, Sulfalen; Glykopeptidantibiotika wieVancomycin, Teicoplanin; Polypeptidantibiotika wie Polymyxine wie Colistin, Polymyxin-B, Nitroimidazol-Derivate wie Metronidazol, Tinidazol; Aminochinolone wie Chloroquin, Mefloquin, Hydroxychloroquin; Biguanide wie Proguanil; Chininalkaloide und Diaminopyrimidine wie Pyrimethamin; Amphenicole wie Chloramphenicol; Rifabutin, Dapson, Fusidinsäure, Fosfomycin, Nifuratel, Telithromycin, Fusafungin, Fosfomycin, Pentamidindiisethionat, Rifampicin, Taurolidin, Atovaquon, Linezolid; Virustatika wie Aciclovir, Ganciclovir, Famciclovir, Foscarnet, Inosin-(Dimepranol-4-acetamidobenzoat), Valganciclovir, Valaciclovir, Cidofovir, Brivudin; antiretrovirale Wirkstoffe (nukleosidanaloge Reverse-Transkriptase-Hemmer und -Derivate) wie Lamivudin, Zalcitabin, Didanosin, Zidovudin, Tenofovir, Stavudin, Abacavir; nicht nukleosidanaloge Reverse-Transkriptase-Hemmer: Amprenavir, Indinavir, Saquinavir, Lopinavir, Ritonavir, Nelfinavir; Amantadin, Ribavirin, Zanamivir, Oseltamivir und Lamivudin, sowie beliebige Kombinationen und Gemische davon.

In besonders bevorzugten Ausführungsformen des erfindungsgemäßen Verfahrens werden als Beschichtungsmaterial in Microcapsules, Liposomen, Nanocapsules, Nanopartikeln, Micellen, synthetischen Phospholipiden, Gas-Dispersionen, Emulsionen, Mikroemulsionen oder Nanospheren inkorporierte pharmakologisch wirksame Stoffe verwendet.

Als Trägermedium für Beschichtungsmateriallösungen, Suspensionen oder Emulsionen können geeignete Lösemittel verwendet werden. Beispiele hierfür sind Methanol, Ethanol, N-Propanol, Isopropanol, Butoxydiglycol, Butoxyethanol, Butoxyisopropanol, Butoxypropanol, n-Butyl-Alkohol, t-Butyl-Alkohol, Butyleneglycol, Butyloctanol, Diethylenglycol, Dimethoxydiglycol, Dimethylether, Dipropylenglycol, Ethoxydiglycol, Ethoxyethanol, Ethylhexandiol, Glycol, Hexanediol, 1,2,6-Hexanetriol, Hexylalkohol, Hexylenglycol, Isobutoxypropanol, Isopentyldiol, 3-Methoxybutanol, Methoxydiglycol, Methoxyethanol, Methoxyisopropanol, Methoxymethylbutanol, Methoxy PEG-10, Methylal, Methyl-Hexylether, Methylpropanediol, Neopentylglycol, PEG-4, PEG-6, PEG-7, PEG-8, PEG-9, PEG-6-Methylether, Pentylenglycol, PPG-7, PPG-2-Buteth-3, PPG-2 Butylether, PPG-3 Butylether, PPG-2 Methylether, PPG-3 Methylether, PPG-2 Propylether, Propanediol, Propylenglycol, Propylenglycol-Butylether, Propylenglycol-Propylether, Tetrahydrofuran, Trimethylhexanol, Phenol, Benzol, Toluol, Xylol; sowie Wasser, ggf. im Gemisch mit Dispersionshilfsmitteln, sowie Mischungen davon.

Nach dem erfindungsgemäßen Verfahren kann die Oberfläche des zu beschichtenden Implantats teilweise, im Wesentlichen vollständig aber auch mehrfach beschichtet werden. Eine Mehrfachbeschichtung erfolgt durch mehrfaches schlupffreies Entlangbewegen der Mantelfläche der Druckwalze und der zu beschichtenden Oberfläche, wobei gegebenenfalls Trocknungsschritte nach jedem Beschichtungsvorgang angewendet werden können.

Besonders bevorzugt ist es, das zu beschichtende Implantat mit einem oder mehreren pharmakologisch wirksamen Stoffen und anschließend mit einer oder mehreren Schichten aus einem oder mehreren, gegebenenfalls unterschiedlichen, die Freisetzung des oder der pharmakologisch wirksamen Stoffe modifizieren Materialien beschichtet wird. Hierfür geeignete freisetzungsmodifizierende Materialien, sind beispielsweise Cellulose und Cellulosederivate wie Hydroxypropylmethylcellulose, Hydroxypropylcellulose, Poly(meth)acrylate, Carbomere, Polyvinylpyrrolidon und dergleichen.

Besonders bevorzugte Ausführungsformen der vorliegenden Erfindung sind beschichtete Gefäßendoprothesen (intraluminale Endoprothesen) wie Stents, Koronarstents, intravaskulare Stents, periphere Stents und dergleichen.

Diese können mit dem erfindungsgemäßen Verfahren auf einfache Weise biokompatibel beaufschlagt werden, wodurch beispielsweise die bei der perkutanen transluminalen Angioplastie mit herkömmlichen Stents häufig auftretenden Restenosen verhindert werden können.

In besonders bevorzugten Ausführungsformen werden Stents, insbesondere mit einer kohlenstoffhaltigen Oberflächenschicht versehene Stents, mit pharmakologisch wirksamen Stoffen oder Stoffgemischen beladen. Beispielsweise können die

Stentoberflächen für die lokale Unterdrückung von Zelladhäsion, Thrombozytenaggregation, Komplementaktivierung bzw. inflammatorische Gewebereaktionen oder Zellproliferation mit folgenden Wirkstoffen ausgerüstet werden:

Heparin, synthetische Heparin-Analoga (z.B. Fondaparinux), Hirudin, Antithrombin III, Drotrecogin alpha, Fibrinolytica (Alteplase, Plasmin, Lysokinasen, Faktor XIIa, Prourokinase, Urokinase, Anistreplase, Streptokinase), Thrombozytenaggregationshemmer (Acetylsalicylsäure, Ticlopidine, Clopidogrel, abciximab, Dextrane), Corticosteroide (Alclometasone, Amcinonide, Augmented Betamethasone, Beclomethasone, Betamethasone, Budesonide, Cortisone, Clobetasol, Clocortolone, Desonide, Desoximetasone, Dexamethasone, Flucinolone, Fluocinonide, Flurandrenolide, Flunisolide, Fluticasone, Halcinonide, Halobetasol, Hydrocortisone, Methylprednisolone, Mometasone, Prednicarbate, Prednisone, Prednisolone, Triamcinolone), sogenannte Non-Steroidal Anti-Inflammatory Drugs (Diclofenac, Diflunisal, Etodolac, Fenoprofen, Flurbiprofen, Ibuprofen, Indomethacin, Ketoprofen, Ketorolac, Meclofenamate, Mefenamic acid, Meloxicam, Nabumetone, Naproxen, Oxaprozin, Piroxicam, Salsalate, Sulindac, Tolmetin, Celecoxib, Rofecoxib), Zytostatika (Alkaloide und Podophyllumtoxine wie Vinblastin, Vincristin; Alkylantien wie Nitrosoharnstoffe, Stickstofflost-Analoga; zytotoxische Antibiotika wie Daunorubicin, Doxorubicin und andere Anthrazykline und verwandte Substanzen, Bleomycin, Mitomycin; Antimetabolite wie Folsäure-, Purin- oder Pyrimidin-Analoga; Paclitaxel, Docetaxel, Sirolimus; Platinverbindungen wie Carboplatin, Cisplatin oder Oxaliplatin; Amsacrin, Irinotecan, Imatinib, Topotecan, Interferon-alpha 2a, Interferon-alpha 2b, Hydroxycarbamid, Miltefosin, Pentostatin, Porfimer, Aldesleukin, Bexaroten, Tretinoin; Antiandrogene, Antiöstrogene).

Für systemische, kardiologische Wirkungen können die Stents erfindungsgemäß beladen werden mit:

Antiarrythmika, insbesondere Antiarrhythmika der Klasse I (Antiarrhythmika vom Chinidintyp: Chinidin, Dysopyramid, Ajmalin, Prajmaliumbitartrat, Detajmiumbitartrat; Antiarrhythmika vom Lidocaintyp: Lidocain, Mexiletin, Phenytoin, Tocainid; Antiarrhythmika der Klasse I C: Propafenon, Flecainid(acetat)), Antiarrhythmika der Klasse II (Betarezeptorenblocker) (Metoprolol, Esmolol, Propranolol, Metoprolol, Atenolol, Oxprenolol), Antiarrhythmika der Klasse III (Amiodaron, Sotalol), Antiarrhythmika der Klasse IV (Diltiazem, Verapamil, Gallopamil), andere Antiarrhythmika wie Adenosin, Orciprenalin, Ipratropiumbromid; Stimulation der Angiogenese im Myokard: Vascular Endothelial Growth Factor (VEGF), Basic Fibroblast Growth Factor (bFGF), nicht virale DNA, virale DNA, endotheliale Wachstumsfaktoren: FGF-1, FGF-2, VEGF, TGF; Antikörper, Monoklonale Antikörper, Anticaline; Stammzellen, Endothelial Progenitor Cells (EPC). Weitere Kardiaka sind: Digitalisglykoside (Acetyldigoxin/Metildigoxin, Digitoxin, Digoxin), weitere Herzglykoside (Ouabain, Proscillaridin). Ferner Antihypertonika (zentral wirksame antiadrenerge Substanzen: Methyldopa, Imidazolinrezeptoragonisten; Kalciumkanalblocker: vom Dihydropyridintyp wie Nifedipin, Nitrendipin; ACE-Hemmer: Quinaprilat, Cilazapril, Moexipril, Trandolapril, Spirapril, Imidapril, Trandolapril; Angiotensin-II-Antagonisten: Candesartancilexetil, Valsartan, Telmisartan, Olmesartanmedoxomil, Eprosartan; peripher wirksame alpha-Rezeptorenblocker: Prazosin, Urapidil, Doxazosin, Bunazosin, Terazosin, Indoramin; Vasodilatatoren: Dihydralazin, Diisopropylamindichloracetat, Minoxidil, Nitroprussidnatrium), andere Antihypertonika wie Indapamid, Co-Dergocrinmesilat, Dihydroergotoxinmethansulfonat, Cicletanin, Bosentan. Weiters Phosphodiesterasehemmer (Milrinon, Enoximon) und Antihypotonika, hier insbesondere adrenerge und dopaminerge Substanzen (Dobutamin, Epinephrin, Etilefrin, Norfenefrin, Norepinephrin, Oxilofrin, Dopamin, Midodrin, Pholedrin, Ameziniummetil), partielle Adrenozeptor-Agonisien (Dihydroergotamin), schließlich andere Antihypotonika wie Fludrocortison.

Für die Steigerung der Gewebeadhäsion, insbesondere bei peripheren Stents können Komponenten der extrazellulären Matrix, Fibronectin, Polylysine, Ethylenevinylacetate, inflammatorische Zytokine wie: TGFβ, PDGF, VEGF, bFGF, TNFα, NGF, GM-CSF, IGF-a, IL-1, IL-8, IL-6, Growth Hormone; sowie adhäsive Substanzen wie: Cyanoacrylate, Beryllium, oder Silica verwendet werden:

Weitere hierfür geeignete Substanzen, systemisch und/oder lokal wirkend, sind Wachstumsfaktoren (Growth Factor), Erythropoetin.

Auch Hormone können in den Stentbeladungen vorgesehen werden, wie-beispielsweise Corticotropine, Gonadotropine, Somatropin, Thyrotrophin, Desmopressin, Terlipressin, Oxytocin, Cetrorelix, Corticorelin, Leuprorelin, Triptorelin, Gonadorelin, Ganirelix, Buserelin, Nafarelin, Goserelin, sowie regulatorische Peptide wie Somatostatin, und/oder Octreotid.

Im Fall von chirurgischen und orthopädischen Implantaten werden häufig Implantate mit makroporösen Oberflächenschichten verwendet. Deren Porengrößen liegen im Bereich von 0,1 bis 1000 µm, bevorzugt bei 1 bis 400 µm, um eine bessere Integration der Implantate durch Einwachsen ins umliegende Zell- oder Knochengewebe zu unterstützen. Diese Implantate eignen sich besonders zur Auftragung und Imprägnierung mit verschiedensten Wirkstoffen und Wirkstoffvorstufen.

Für orthopädische und nichtorthopädische Implantate sowie Herzklappen, Herzschrittmacherelektroden oder Kunstherzteile können ferner für die lokale

Unterdrückung von Zelladhäsion, Thrombozytenaggregation, Komplementaktivierung bzw. inflammatorische Gewebereaktion oder Zellproliferation im Wesentlichen die gleichen Wirkstoffe eingesetzt werden wie in den oben beschriebenen Stentanwehdungen.

Ferner können zur Stimulation von Gewebewachstum insbesondere bei orthopädischen Implantaten für eine bessere Implantatintegration folgende Wirkstoffe verwendet werden: Bone and Cartilage Stimulating Peptides, bone morphogenetic proteins (BMPs), insbesondere rekombinante BMP's (z.B. Recombinant human BMP-2 (rhBMP-2)), Bisphosphonate (z.B. Risedronate, Pamidronate, Ibandronate, Zoledronsäure, Clodronsäure, Etidronsäure, Alendronsäure, Tiludronsäure), Fluoride (Dinatriumfluorophosphat, Natriumfluorid); Calcitonin, Dihydrotachystyrol. Dann alle Growth Factors und Zytokine (Epidermal Growth Factor (EGF), Platelet-Derived Growth Factor (PDGF), Fibroblast Growth Factors (FGFs), Transforming Growth Factors-b TGFs-b), Transforming Growth Factor-a (TGF-a), Erythropoietin (Epo), Insulin-Like Growth Factor-I (IGF-I), Insulin-Like Growth Factor-II (IGF-II), Interleukin-1 (IL-1), Interleukin-2 (IL-2), Interleukin-6 (IL-6), Interleukin-8 (IL-8), Tumor Necrosis Factor-a (TNF-a), Tumor Necrosis Eactor-b (TNE-b), Interferon-g (INF-g), Colony Stimulating Factors (CSFs)). Weitere adhäsions- und integrationsfördernde Substanzen sind neben den bereits genannten inflammatorischen Cytokinen das Monocyte chemotactic protein, fibroblast stimulating factor 1, Histamin, Fibrin oder Fibrinogen, Endothelin-1, Angiotensin II, Kollagene, Bromocriptin, Methylsergid, Methotrexat, Kohlenstofftetrachlorid, Thioacetamid, Ethanol.

Darüber hinaus können die Implantate mit dem erfindungsgemäßen Druckauftragsverfahren auch mit antibakteriellen-antiinfektiösen Beschichtungen versehen werden, wobei die folgenden Stoffe oder Stoffgemische als Beschichtungsmaterial verwendbar sind: Silber(ionen), Titandioxid, Antibiotika und Antiinfektiva. Insbesondere beta-Laktam-Antibiotika (β-Lactam-Antibiotika: β-Lactamase-sensitive Penicilline wie Benzylpenicilline (Penicillin G), Phenoxymethylpenicillin (Penicillin V); β-Lactamase-resistente Penicilline wie Aminopenicilline wie Amoxicillin, Ampicillin, Bacampicillin; Acylaminopenicilline wie Mezlocillin, Piperacillin; Carboxypenicilline, Cephalosporine (Cefazolin, Cefuroxim, Cefoxitin, Cefotiam, Cefaclor, Cefadroxil, Cefalexin, Loracarbef, Cefixim, Cefuroximaxetil, Ceftibuten, Cefpodoximproxetil, Cefpodoximproxetil) oder andere wie Aztreonam, Ertapenem, Meropenem. Weitere Antibiotika sind β-Lactamase-Inhibitoren (Sulbactam, Sultamicillintosilat), Tetracycline (Doxycyclin, Minocyclin, Tetracyclin, Chlortetracyclin, Oxytetracyclin), Aminoglykoside (Gentamicin, Neomycin, Streptomycin, Tobramycin, Amikacin, Netilmicin, Paromomycin, Framycetin, Spectinomycin), Malaolidantibiotika (Azithromycin, Clarithromycin, Erythromycin, Roxithromycin, Spiramycin, Josamycin), Lincosamide (Clindamycin, Lincomycin), Gyrasehemmer (Fluorochinolone wie Ciprofloxacin, Ofloxacin, Moxifloxacin, Norfloxacin, Gatifloxacin, Enoxacin, Fleroxacin, Levofloxacin; andere Chinolone wie Pipemidsäure), Sulfonamide und Trimethoprim (Sulfadiazin, Sulfalen, Trimethoprim), Glykopeptidantibiotika (Vancomycin, Teicoplanin), Polypeptidantibiotika (Polymyxine wie Colistin, Polymyxin-B), Nitroimidazol-Derivate (Metronidazol, Tinidazol), Aminochinolone (Chloroquin, Mefloquin, Hydroxychloroquin), Biguanide (Proguanil), Chininalkaloide und Diaminopyrimidine (Pyrimethamin), Amphenicole (Chloramphenicol) und andere Antibiotika (Rifabutin, Dapson, Fusidinsäure, Fosfomycin, Nifuratel, Telithromycin, Fusafungin, Fosfomycin, Pentamidindiisethionat, Rifampicin, Taurolidin, Atovaquon, Linezolid). Unter den Virustatika sind zu nennen Aciclovir, Ganciclovir, Famciclovir, Foscarnet, Inosin-(Dimepranol-4-acetamidobenzoat), Valganciclovir, Valaciclovir, Cidofovir, Brivudin. Dazu zählen auch Antiretrovirale Wirkstoffe (nukleosidanaloge Reverse-Transkriptase-Hemmer und -Derivate: Lamivudin, Zalcitabin, Didanosin, Zidovudin, Tenofovir, Stavudin, Abacavir; nicht nukleosidanaloge Reverse-Transkriptase-Hemmer: Amprenavir, Indinavir, Saquinavir, Lopinavir, Ritonavir, Nelfinavir) und andere Virustatika wie Amantadin, Ribavirin, Zanamivir, Oseltamivir, Lamivudin.

In besonders bevorzugten Ausführungsformen der vorliegenden Erfindung können die Implantate mittels weiterer Agenzien in ihren chemischen oder physikalischen Eigenschaften geeignet modifiziert werden, beispielsweise um die Hydrophilie, Hydrophobie, elektrische Leitfähigkeit, Haftung oder sonstige Oberflächeneigenschaften zu modifizieren. Hierfür als Beschichtungsmaterial einsetzbare Stoffe sind biodegradierbare oder nicht-degradierbare Polymere, wie beispielsweise bei den biodegradierbaren: Kollagene, Albumin, Gelatin, Hyaluronsäure, Stärke, Cellulose (Methylcellulose, Hydroxypropylcellulose, Hydroxypropylmethylcellulose, Carboxymethylcellulose-Phtalat; weiterhin Kasein, Dextrane, Polysaccharide, Fibrinogen, Poly(D,L-Lactide), Poly(D,L-Lactide-Co-Glycolide), Poly(Glycolide), Poly(Hydroxybutylate), Poly(Alkylcarbonate), Poly(Orthoester), Polyester, Poly(Hydroxyvaleric Acid), Polydioxanone, Poly(Ethylen-Terephtalate), Poly(malatsäure), Poly(Tartronsäure), Polyanhydride, Polyphosphohazene, Poly(Aminosäuren), und alle ihre Co-Polymere.

Zu den nicht-biodegradierbaren zählen: Poly(Ethylen-Vinylacetate), Silicone, Acrylpolymere wie Polyacrylsäure, Polymethylacrylsäure, Polyacrylcynoacrylat; Polyethylene, Polypropylene, Polyamide, Polyurethane, Poly(Ester-Urethane), Poly(Ether-Urethane), Poly(Ester-Harnstoffe), Polyether wie Polyethylenoxid, Polypropylenoxid, Pluronics, PolyTetramethylenglycol; Vinylpolymere wie Polyvinylpyrrolidone, Poly(Vinyl-alkohole), Poly(vinyl-acetat-phatalat).

Generell gilt, dass Polymere mit anionischen (z.B. Alginat, Carrageenan, carboxymethylcellulose) oder kationischen (z.B. Chitosan, Poly-L-Lysine etc.) oder beiden Eigenschaften (Phoshporylcholin) vorteilhaft verwendet werden können.

Zur Modifizierung der Freisetzungseigenschaften wirkstoffhaltiger erfindungsgemäß beschichteter Implantate können durch Auftragen beispielsweise von weiteren Polymeren spezifische pH- oder temperaturabhängige Freisetzungseigenschaften erzeugt werden. PH-sensitive Polymere sind beispielsweise Poly(Acrylsäure) und Derivate, zum Beispiel: Homopolymere wie Poly(Aminocarboxylsäure), Poly(Acrylsäure), Poly(Methyl-Acrylsäure) und deren Co-Polymere. Ebenso gilt dies für Polysaccharide wie Celluloseacetat-Phtalat, Hydroxypropylinethylcellulose-Phtalat, Hydroxypropylmethylcellulose-Succinat, Celluloseacetat-Trimellitat und Chitosan. Thermosensitive Polymere sind beispielsweise Poly(N-Isopropylacrylamid-Co-Natrium-Acrylat-Co-n-N-Alkylacrylamid), Poly(N-Methyl-N-n-propylacrylamid), Poly(N-Methyl-N-Isopropylacrylamid), Poly(N-n-Propylmethacrylamid), Poly(N-Isopropylacrylamid), Poly(N,n-Diethylacrylamid), Poly(N-Isopropylmethacrylamid), Poly(N-Cyclopropylacrylamid), Poly(N-Ethylacrylamid), Poly(N-Ethylmethyacrylamid), Poly(N-Methyl-N-Ethylacrylamid), Poly(N-Cyclopropylacrylamid). Weitere Polymere mit Thermogel-Charakteristik sind Hydroxypropyl-Cellulose, Methyl-Cellulose, Hydroxypropylmethyl-Cellulose, Ethylhydroxyethyl-Cellulose und Pluronics wie F-127, L-122, L-92, L-81, L-61.

Bei der zusätzlichen Beschichtung der erfindungsgemäß beladenen Implantate kann daher zwischen physischen Barrieren wie inerten biodegradierbaren Substanzen (Poly-1-Lysin, Fibronectin, Chitosan, Heparin etc.) und biologisch aktiven Barrieren unterschieden werden. Letztere können sterisch behindernde Moleküle sein, die physiologisch bioaktiviert werden und die Freisetzung von Wirkstoffen bzw. deren Trägern gestatten. Beispielsweise Enzyme, welche die Freisetzung vermitteln, biologisch aktive Stoffe aktivieren oder nicht-aktive Beschichtungen binden und zur Exposition von Wirkstoffen führen.

Die erfindungsgemäß beschichteten Implantate können in besonderen Anwendungen auch mit lebenden Zellen oder Mikroorganismen beladen werden. Diese können sich in geeignet porösen Oberflächen der Implantate ansiedeln, wobei das so besiedelte Implantat dann mit einem geeigneten Membran bzw. membranartigen Überzug versehen werden kann, der für Nährstoffe und von den Zellen oder Mikroorganismen erzeugte Wirkstoffe durchlässig ist, nicht jedoch für die Zellen selbst.

Auf diese Weise lassen sich unter Anwendung der erfindungsgemäßen Technologie durch bedrucken mit Suspensionen von insulinproduzierenden Zellen beispielsweise Implantate herstellen, die insulinproduzierende Zellen enthalten, welche nach Implantierung im Körper in Abhängigkeit vom Glukosespiegel der Umgebung Insulin produzieren und freisetzen.

Im Folgenden wird beispielhaft eine bevorzugte Ausführungsform des erfindungsgemäßen Verfahrens und der erfindungsgemäßen Vorrichtung zum Auftragen von Wirkstoffen auf die Oberfläche von Stents beschrieben. Diese Ausführungen zur Erläuterung einer beispielhaften Ausführungsform sind lediglich zur weiteren Veranschaulichung der erfindungsgemäßen Prinzipien gedacht und stellen keine Einschränkung des allgemeinen Erfindungsgedankens auf eine bestimmte Ausführungsform dar.

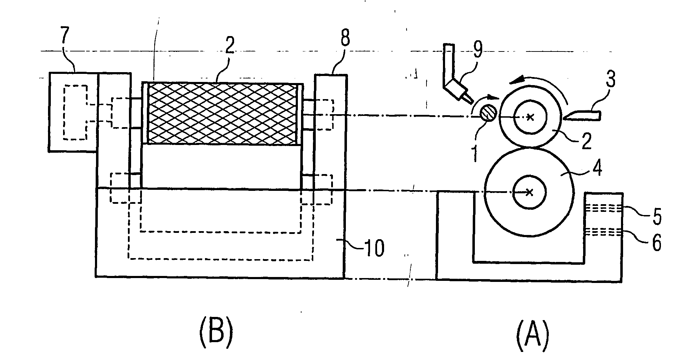

Figur 1 zeigt zwei Ansichten **A** und **B** einer Ausführungsform der erfindungsgemäßen Vorrichtung zum Auftragen einer definierten Menge eines Beschichtungsmaterials auf die Oberfläche eines zu beschichtenden Implantats mittels einer Druckwalze.

Bei 1 gezeigt ist ein Implantat angeordnet, hier ein zylindrischer Stent, der auf einer Antriebswelle angeordnet ist, die gegen die Druckwalze **2** schlupffrei angetrieben werden kann. Die Druckwalze **2** ist in der Ausführungsform der Figur 1 eine Präzisionsrasterwalze, mit einem Antrieb **7** welcher eine schlupffreie Bewegung in Bezug auf die Antriebswelle des Stents **1** in zur Präzisionsrastewalze **2** gegenläufiger Bewegung ermöglicht. In der bevorzugten Ausführungsform der Vorrichtung gemäß Figur 1 erfolgt die Übertragung des Beschichtungsmaterials von der Präzisionsrasterwalze **2** auf den Stent **1** kontaktfrei.

Wie in der Seitenansicht **A** der Figur 1 zu sehen befindet sich die Präzisionsrasterwalze **2** in direktem Kontakt mit einer Schöpfwalze **4,** welche zumindest teilweise in einen Vorrats-behälter **10** eintaucht, die mit Beschichtungsmaterial oder Beschichtungsmateriallösung befüllt ist. Die Bewegung der Schöpfwalze **4** erfolgt gegenläufig zur Präzisionsrasterwalze **2.** Der Füllstand des Beschichtungsmaterials in dem Vorratsbehälter **10** kann wie in der Seitenansicht **A** der Figur 1 angegeben durch Füllstandssensoren **5** und **6** zur Bestimmung des oberen und unteren Füllstands im Vorratsbehälter ausgestattet sein. Die Füllstandssensoren **5** und **6** können beispielsweise kapazitive oder Leitfähigkeitssensoren sein, und sie ermöglichen im automatisierten Betrieb ein regelmäßiges Nachfüllen des Vorratsbehälters **10** mit Beschichtungsmaterial, wobei das Niveau des Beschichtungsmaterials in dem Vorratsbehälter zwischen den durch die Füllstandssensoren **5** und **6** vorgegebenen Füllhöhen mittels einer geeigneten automatischen Steuerung gehalten wird.

Das von der Schöpfwalze **4** aufgenommene Beschichtungsmaterial wird durch Kontakt auf die Präzisionsrasterwalze **2** übertragen, wobei die Vertiefungen in der Rasterwalze mit-Beschichtungsmaterial befüllt werden. Überschüssiges Beschichtungsmaterial auf der Präzisionsrasterwalze **2** wird mit einer Abstreifvorrichtung **3** abgestrichen, wie beispielsweise ein Messerbalken, um eine durch das Volumen der Vertiefungen der Präzisionsrasterwalze **2** vorgegebene definierte Beschichtungsmaterialmenge zu erhalten. Die Präzisionsrasterwalze **2** wird gegenläufig zur Stentantriebswelle **1** schlupffrei rotiert, so dass durch die Zahl der Umdrehungen definiert eine bestimmte Menge an Beschichtungsmaterials bei jeder vollständigen Umdrehung des Stents **1** von der Präzisionsrasterwalze **2** auf den Stent **1** übertragen wird. Im kontaktfreien Verfahren geschieht dies durch Übergang des Beschichtungsmaterials von der Präzisionsrasterwalze **2** auf den Stent **1** infolge der der Oberflächenbeschaffenheit des zu beschichtenden Stents immanenten Adsorptions-bzw. Adhäsionskräfte, welche durch geeignetes Anordnen der Druckwalze **2** bei dem zu beschichtenden Stent **1** ausreichen um das in den Vertiefungen der Mantelfläche der Druckwalze **2** befindliche Beschichtungsmaterial anziehen zu können.

Wie in der Frontalansicht **B** zu sehen, wird der Stent **1** auf einer Welle in Wellenlagerblöcken **8** gehalten und die Stentwelle **1** bzw. Rasterwalze 2 über einen entsprechenden Präzisionsantrieb 7 schlupffrei gegeneinander bewegt.

In der beschriebenen Ausführungsform der Figur 1 sind die Wellenlagerblöcke **8** in einem Gehäuse untergebracht, welches als integrales Bauteil auch den Wirkstoffvorratsbehälter **10** vorgesehen ist, wodurch sich eine kompakte Bauweise ergibt.

In einer besonders bevorzugten Ausführungsform der vorliegenden Erfindung kann in räumlicher Nähe zum Stent **1** eine geeignete Trockungsvorrichtung **9** vorgesehen sein, wie beispielsweise eine Luftdüse zum Beströmen des Stents mit erhitztem Inertgas, um Lösungsmittel zu verdampfen oder das Beschichtungsmaterial zu trocknen. Alternativ hierzu kann die Trocknungsvorrichtung **9** auch eine Wärmestrahlungsvorrichtung sein, wie beispielsweise eine Infrarotlampe oder dergleichen.

## Patentansprüche

1. Verfahren zum Auftragen einer definierten Menge eines Beschichtungsmaterials auf die Oberfläche eines zu beschichtenden medizinischen Implantats mittels eines Druckverfahrens, umfassend die Schritte:
- Beladen der in der Mantelfläche einer Druckwalze ausgebildeten Vertiefungen mit einer definierten Menge des Beschichtungsmaterials;
- Anordnen der Druckwalze bei dem zu beschichtenden Implantat in der Weise, dass die der Oberflächenbeschaffenheit des zu beschichtenden Implantats immanenten Adsorptions- bzw. Adhäsionskräfte ausreichen, um das in den Vertiefungen der Mantelfläche der Druckwalze befindliche Beschichtungsmaterial anziehen zu können;
- Auftragen des in den Vertiefungen der Mantelfläche der Druckwalze befindlichen Beschichtungsmaterials durch Entlangbewegen der Mantelfläche der Druckwalze und der Oberfläche des zu beschichtenden Implantats.

2. Verfahren nach Anspruch 1,
**dadurch gekennzeichnet, dass** die in der Mantelfläche der Druckwalze ausgebildeten Vertiefungen mit einer definierten Menge Beschichtungsmaterial beladen werden, indem die Vertiefungen mit Beschichtungsmaterial befüllt werden und anschließend mögliche Beschichtungsmaterialüberschüsse von der Mantelfläche entfernt werden.

3. Verfahren nach Anspruch 1 oder 2,
**dadurch gekennzeichnet, dass** das Beladen der in der Mantelfläche der Druckwalze ausgebildeten Vertiefungen mit Beschichtungsmaterial über eine rotierende Schöpfwalze erfolgt, wobei sich während der Rotation zumindest ein Zylindersegment der Schöpfwalze ständig in dem Beschichtungsmaterialbad befindet, wodurch die Schöpfwalze umfangsseitig mit Beschichtungsmaterial benetzt wird, und indem die Schöpfwalze das so aufgenommene Beschichtungsmaterial auf die Druckwalze überträgt.

4. Verfahren nach einem der Ansprüche 1 bis 3,
**dadurch gekennzeichnet, dass** die Positionierung der Druckwalze zu dem zu beschichtenden Implantat direkten Kontakt ermöglicht.

5. Verfahren nach einem der Ansprüche 1 bis 3,
**dadurch gekennzeichnet, dass** die Positionierung der Druckwalze zu dem zu beschichtenden Implantat kontaktfrei erfolgt.

6. Verfahren nach einem der Ansprüche 1 bis 5,
**dadurch gekennzeichnet, dass** Entlangbewegen der Mantelfläche der Druckwalze und der Oberfläche des zu beschichtenden Implantats im wesentlichen schlupffrei erfolgt.

7. Verfahren nach einem der Ansprüche 1 bis 6,
**dadurch gekennzeichnet, dass** das Entlangbewegen der Mantelfläche der Druckwalze und der Oberfläche des zu beschichtenden Implantats erfolgt, indem die Druckwalze und das zu beschichtende Implantat um zwei im wesentlichen zueinander parallel liegende Achsen gegensinnig gedreht werden.

8. Verfahren nach einem der Ansprüche 1 bis 6,
**dadurch gekennzeichnet, dass** das schlupffreie Entlangbewegen der Mantelfläche der Druckwalze und der Oberfläche des zu beschichtenden Implantats erfolgt, indem die Achse der Druckwalze äquidistant zu der Oberfläche des zu beschichtenden Implantats entlang bewegt wird.

9. Verfahren nach einem der vorhergehenden Ansprüche,
**dadurch gekennzeichnet, dass** das zu beschichtende Implantat ausgewählt ist aus medizinischen oder therapeutischen Implantaten wie Gefäßendoprothesen, Stents, Koronarstents, peripheren Stents, orthopädischen Implantaten, Knochen- oder Gelenkprothesen, Kunstherzen, künstliche Herzklappen, Herzschrittmacher-elektroden, subkutane und/oder intramuskuläre Implantate und dergleichen.

10. Verfahren nach Anspruch 9,
**dadurch gekennzeichnet, dass** das zu beschichtende Implantat ein Stent ist, insbesondere ein kohlenstoffbeschichteter Stent.

11. Verfahren nach einem der vorhergehenden Ansprüche,
**dadurch gekennzeichnet, dass** die Druckwalze ausgewählt ist aus Gravurwalzen, Rasterwalzen, Tiefdruckwalzen, Keramikwalzen, Keramikrasterwalzen, keramikbeschichtete Rasterwalzen, Flexodruckwalzen, Prägewalzen, Kalanderwalzen, sowie sonstiger Druckwalzen, deren Mantelflächen Vertiefungen zur Aufnahme von Beschichtungsmaterial aufweisen, besonders bevorzugt Rasterwalzen.

12. Verfahren nach einem der vorhergehenden Ansprüche,
**dadurch gekennzeichnet, dass** das Beschichtungsmaterial eine Lösung, Suspension oder Emulsion eines oder mehrerer Wirkstoffe oder Wirkstoffvorstufen in einem geeigneten Trägermedium ist.

13. Verfahren nach Anspruch 12,
**dadurch gekennzeichnet, dass** die Wirkstoffe oder Wirkstoffvorstufen ausgewählt sind aus pharmakologisch wirksamen Stoffen, Mikroorganismen, lebendem organischen Zellmaterial, sowie biologisch verträglichen anorganischen oder organischen Stoffen.

14. Verfahren nach Anspruch 13,
**dadurch gekennzeichnet, dass** die pharmakologisch wirksamen Stoffe in Microcapsules, Liposomen, Nanocapsules, Nanopartikeln, Micellen, synthetischen Phospholipiden, Gas-Dispersionen, Emulsionen, Mikroemulsionen, oder Nanospheres inkorporiert werden.

15. Verfahren nach einem der vorhergehenden Ansprüche,
**dadurch gekennzeichnet, dass** die Oberfläche des zu beschichtenden Implantats teilweise, im wesentlichen vollständig und/oder mehrfach beschichtet wird.

16. Verfahren nach Anspruch 15,
**dadurch gekennzeichnet, dass** das Implantat mit einer Schicht aus einer oder mehreren pharmakologisch wirksamen Stoffen und anschließend mit einer oder mehreren Schichten aus einem oder mehreren, ggf. unterschiedlichen, die Freisetzung des oder der pharmakologisch wirksamen Stoffe modifizierenden Materialien beschichtet wird.

17. Vorrichtung zum Auftragen einer definierten Menge eines Beschichtungsmaterials auf die Oberfläche eines zu beschichtenden Implantats mit einer Druckwalze, in deren Mantelfläche eine Vielzahl an Vertiefungen ausgebildet sind, um eine definierte Menge an Beschichtungsmaterial aufnehmen zu können, wobei die Druckwalze stets bei dem zu beschichtenden Implantat so angeordnet ist, dass die der Oberflächenbeschaffenheit des zu beschichtenden Implantats immanenten Saug- oder Adsorptionskräfte ausreichen, um das in den Vertiefungen der Mantelfläche der Druckwalze befindliche Beschichtungsmaterial anziehen zu können, um durch schlupffreies Entlangbewegen der Mantelfläche der Druckwalze und der Oberfläche des zu beschichtenden Körpers das in den Vertiefungen der Mantelfläche der Druckwalze befindliche Beschichtungsmaterial auf die Oberfläche des zu beschichtenden Implantats aufzutragen.

18. Verwendung eines Druckverfahrens zum Auftragen einer definierten Menge eines Beschichtungsmaterials mit einer Rasterwalze auf ein zu beschichtendes Implantat.

19. Verwendung nach Anspruch 18,
**dadurch gekennzeichnet, dass** das zu beschichtende Implantat ausgewählt ist aus medizinischen oder therapeutischen Implantaten wie Gefäßendoprothesen, Stents, Koronarstents, peripheren Stents, orthopädischen Implantaten, Knochen- oder Gelenkprothesen, Kunstherzen, künstliche Herzklappen, Herzschrittmacher-elektroden, subkutane und/oder intramuskuläre Implantate und dergleichen.

20. Verwendung nach einem der Anspüche 18 oder 19,
**dadurch gekennzeichnet, dass** das Beschichtungsmaterial eine Lösung, Suspension oder Emulsion eines oder mehrerer Wirkstoffe oder Wirkstoffvorstufen in einem geeigneten Trägermedium ist.

21. -Verwendung nach Anspruch 20,
**dadurch gekennzeichnet, dass** die Wirkstoffe oder Wirkstoffvorstufen ausgewählt sind aus pharmakologisch wirksamen Stoffen, Mikroorganismen, lebendem organischen Zellmaterial, sowie biologisch verträglichen anorganischen oder organischen Stoffen.

22. Beschichtes Implantat, herstellbar nach dem Verfahren nach einem der Ansprüche 1 bis 16.

## Claims

1. A method for applying a defined amount of a coating material onto the surface of a medical implant to be coated by means of a printing method comprising the steps of:
- charging the recesses formed in the jacket surface of a printing roller with a defined amount of the coating material;
- arranging the printing roller at the implant to be coated in such a way that the adsorption and/or adhesion forces which are intrinsic to the surface properties of the implant to be coated suffice to be able to attract the coating material present in the recesses of the jacket surface of the printing roller;
- applying the coating material present in the recesses of the jacket surface of the printing roller by moving along the jacket surface of the printing roller and the surface of the implant to be coated.

2. The method of claim 1,
**characterised in that** the recesses formed in the jacket surface of the printing roller are charged with a defined amount of coating material by filling the recesses with coating material, and subsequently removing possible coating material excesses from the jacket surface.

3. The method of claim 1 or 2,
**characterised in that** charging of the recesses formed in the jacket surface of the printing roller is effected with coating material via a rotating scoop roller, at least one cylinder segment of the scoop roller being continually present in the coating material bath during rotation, as a result of which the scoop roller is wetted circumferencially with coating material and the scoop roller transferring the coating material thus taken up onto the printing roller.

4. The method of any one of claims 1 to 3,
**characterised in that** the positioning of the printing roller vis-à-vis the coated implant allows direct contact.

5. The method of any one of claims 1 to 3,
**characterised in that** the positioning of the printing roller vis-à-vis the coated implant takes place in a contact-free manner.

6. The method of any one of claims 1 to 5,
**characterised in that** the moving along of the jacket surface of the printing roller and the surface of the implant to be coated takes place in an essentially slip-free manner.

7. The method of any one of claims 1 to 6,
**characterised in that** moving along of the jacket surface of the printing roller and the surface of the coated implant takes place by the printing roller and the implant to be coated being rotated in the opposite direction around two essentially parallel axes.

8. The method of any one of claims 1 to 6,
**characterised in that** the slip-free moving along of the jacket surface of the printing roller and the surface of the implant to be coated takes place by moving the axis of the printing roller in an equidistant manner to the surface of the implant to be coated.

9. The method of one of the preceding claims,
**characterised in that** the implant to be coated is selected from medical or therapeutic implants such as vascular endoprostheses, stents, coronary stents, peripheral stents, orthopaedic implants, bone or joint prostheses, artificial hearts, artificial heart valves, pacemaker electrodes, subcutaneous and/or intramuscular implants, and the like.

10. The method of claim 9,
**characterised in that** the implant to be coated is a stent, in particular a carbon-coated stent.

11. The method of any one of the preceding claims,
**characterised in that** the printing roller is selected from from gravure rollers, anilox rollers, rotogravure rollers, ceramic rollers, ceramic anilox rollers, ceramic-coated anilox rollers, flexographic printing rollers, embossing rollers, calendar rollers and other printing rollers whose jacket surfaces exhibit recesses for receiving coating material.

12. The method of any one of the preceding claims,
**characterised in that** the coating material is a solution, suspension or emulsion of one or several active agents or active agent precursors in a suitable carrier medium.

13. The method of claim 12,
**characterised in that** the active agents or active agent precursors are selected from pharmacologically effective substances, micro-organisms, living organic cell material as well as biocompatible inorganic or organic substances.

14. The method of claim 13,
**characterised in that** the pharmacologically effective substances are incorporated into microcapsules, liposomes, nanocapsules, nanoparticles, micelles, synthetic phospholipids, gas dispersions, emulsions, micro-emulsions or nanospheres.

15. The method of any one of the preceding claims,
**characterised in that** the surface of the implant to be coated is coated partially, essentially completely and/or multiply.

16. The method of claim 15,
**characterised in that** the implant is coated with a layer of one or several pharmacologically effective substances and subsequently with one or several layers of one or several, if necessary, different materials modifying the release of the pharmacologically effective substance or substances.

17. A device for applying a defined amount of a coating material onto the surface of an implant to be coated using a printing roller in whose jacket surface a plurality of recesses have been formed in order to be able to take up a defined amount of a coating material, the printing roller being arranged at the implant to be coated in such a way that the suction and/or adsorption forces which are intrinsic to the surface properties of the implant to be coated suffice to be able to attract the coating material present in the recesses of the jacket surface of the printing roller, in order to apply, by moving along, in a slip-free manner, the jacket surface of the printing roller and the surface of the body to be coated, the coating material present in the recesses of the jacket surface of the printing roller onto the surface of the implant to be coated.

18. A use of a printing process for applying a defined amount of a coating material onto an implant to be coated with an anilox roller.

19. The use of claim 18,
**characterised in that** the implant to be coated is selected from medical or therapeutic implants such as vascular endoprostheses, stents, coronary stents, peripheral stents, orthopaedic implants, bone or joint prostheses, artificial hearts, artificial heart valves, pacemaker electrodes, subcutaneous and/or intramuscular implants and the like.

20. The use of any one of claims 18 or 19,
**characterised in that** the coating material is a solution, suspension or emulsion of one or several active agents or active agent precursors in a suitable carrier medium.

21. The use of claim 20,
**characterised in that** the active agents or active agent precursors are selected from pharmacologically effective substances, micro-organisms, living organic cell material as well as biocompatible inorganic or organic substances.

22. A coated implant, producible according to the method of any one of claims 1 to 16.

## Revendications

1. Procédé d'application d'une quantité définie d'un matériau de revêtement sur la surface d'un implant médical à recouvrir au moyen d'un procédé d'impression, comprenant les étapes :
- chargement des creux formés dans la surface latérale d'un cylindre d'impression avec une quantité définie de matériau de revêtement ;
- disposition du cylindre d'impression près de l'implant à recouvrir de telle manière que les forces d'adsorption et d'adhésion immanentes à l'état de surface de l'implant à recouvrir soient suffisantes pour pouvoir attirer le matériau de revêtement se trouvant dans les creux de l'enveloppe latérale du cylindre d'impression ;
- application du matériau de revêtement se trouvant dans les creux de la surface latérale du cylindre d'impression par déplacement le long l'un de l'autre de la surface latérale du cylindre d'impression et de la surface de l'implant à recouvrir.

2. Procédé selon la revendication 1, **caractérisé en ce que** les creux formés dans la surface latérale du cylindre d'impression sont chargés d'une quantité définie de matériau de revêtement en remplissant les creux avec le matériau de revêtement et ensuite en éliminant de la surface latérale des excédents de matériau de revêtement possibles.

3. Procédé selon la revendication 1 ou la revendication 2, **caractérisé en ce que** le chargement en matériau de revêtement des creux formés dans la surface latérale du cylindre d'impression est effectué par l'intermédiaire d'un cylindre barboteur rotatif, au moins un segment cylindrique du rouleau barboteur se trouvant pendant la rotation dans le bain de matériau de revêtement, le rouleau barboteur étant ainsi mouillé par le matériau de revêtement côté circonférence, et le cylindre barboteur transmettant le matériau de revêtement ainsi repris au cylindre d'impression.

4. Procédé selon l'une des revendications 1 à 3, **caractérisé en ce que** le positionnement du cylindre d'impression par rapport à l'implant à revêtir permet le contact direct.

5. Procédé selon l'une des revendications 1 à 3, **caractérisé en ce que** le positionnement du cylindre d'impression par rapport à l'implant à recouvrir a lieu sans contact.

6. Procédé selon l'une des revendications 1 à 5, **caractérisé en ce que** le mouvement le long l'un de l'autre de la surface latérale du cylindre d'impression et de la surface de l'implant à recouvrir a lieu essentiellement sans glissement.

7. Procédé selon l'une des revendications 1 à 6, **caractérisé en ce que** le mouvement le long l'un de l'autre de la surface latérale du cylindre d'impression et de la surface de l'implant à recouvrir a lieu par rotation en sens contraire du cylindre d'impression et de l'implant à recouvrir autour de deux axes situés essentiellement parallèlement l'un à l'autre.

8. Procédé selon l'une des revendications 1 à 6, **caractérisé en ce que** le mouvement le long l'un de l'autre sans glissement de la surface latérale du cylindre d'impression et de la surface de l'implant à recouvrir est effectué par déplacement de l'axe du cylindre d'impression le long de la surface de l'implant à recouvrir et de manière équidistante à celle-ci.

9. Procédé selon l'une quelconque des revendications précédentes, **caractérisé en ce que** l'implant à recouvrir est choisi parmi des implants médicaux ou thérapeutiques, tels que des endoprothèses vasculaires, stents, stents coronariens, stents périphériques, implants orthopédiques, prothèses osseuses ou articulaires, coeurs artificiels, valves cardiaques artificielles, électrodes de stimulateur cardiaque, implants sous-cutanés et/ou intramusculaires et similaires.

10. Procédé selon la revendication 9, **caractérisé en ce que** l'implant à recouvrir est un stent, en particulier un stent recouvert de carbone.

11. Procédé selon l'une quelconque des revendications précédentes, **caractérisé en ce que** le cylindre d'impression est choisi parmi des cylindres gravés, cylindres tramés, cylindres d'impression en héliogravure, cylindres en céramique, cylindres tramés en céramique, cylindres tramés revêtus de céramique, cylindres d'impression flexographique, cylindres de gaufrage, cylindres de calandrage, ainsi que d'autres cylindres d'impression dont la surface latérale comporte des creux pour recevoir du matériau de revêtement, en particulier de préférence des cylindres tramés.

12. Procédé selon l'une quelconque des revendications précédentes, **caractérisé en ce que** le matériau de revêtement est une solution, suspension ou émulsion d'une ou plusieurs substances actives ou un état préalable de substance active dans un milieu support approprié.

13. Procédé selon la revendication 12, **caractérisé en ce que** les substances actives ou états préalables de substances actives sont choisis parmi des substances pharmacologiquement actives, micro-organismes, matière cellulaire organique vivante, ainsi que des substances organiques ou anorganiques compatibles biologiquement.

14. Procédé selon la revendication 13, **caractérisé en ce que** les substances pharmacologiquement actives sont incorporées dans des microcapsules, liposomes, nanocapsules, nanoparticules, micelles, phospholipides synthétiques, dispersions gazeuses, émulsions, microémulsions ou nanosphères.

15. Procédé selon l'une quelconque des revendications précédentes, **caractérisé en ce que** la surface de l'implant à recouvrir est recouverte en partie, essentiellement en totalité et/ou plusieurs fois.

16. Procédé selon la revendication 15, **caractérisé en ce que** l'implant est recouvert par une couche d'une ou plusieurs substances pharmacologiquement actives et ensuite par une ou plusieurs couches d'un ou plusieurs matériaux éventuellement différents, modifiant la libération de la ou des substances pharmacologiquement actives.

17. Dispositif pour l'application d'une quantité définie d'un matériau de revêtement sur la surface d'un implant à recouvrir, avec un cylindre d'impression dans la surface latérale duquel une pluralité de creux sont formés pour pouvoir recevoir une quantité définie du matériau de revêtement, le cylindre d'impression étant toujours disposé près de l'implant à recouvrir, de telle manière que les forces d'aspiration ou d'adsorption immanentes à l'état de surface de l'implant à recouvrir suffisent pour pouvoir attirer le matériau de revêtement se trouvant dans les creux de la surface latérale du cylindre d'impression pour, par déplacement le long l'un de l'autre sans glissement de la surface latérale du cylindre d'impression et de la surface du corps à recouvrir, appliquer le matériau de revêtement se trouvant dans les creux de la surface latérale du cylindre d'impression sur la surface de l'implant à recouvrir.

18. Utilisation d'un procédé d'impression pour appliquer une quantité définie d'un matériau de revêtement avec un cylindre tramé sur un implant à recouvrir.

19. Utilisation selon la revendication 18, **caractérisée en ce que** l'implant à recouvrir est choisi parmi des implants médicaux ou thérapeutiques, tels que des endoprothèses vasculaires, stents, stents coronariens, stents périphériques, implants orthopédiques, prothèses osseuses ou articulaires, coeurs artificiels, valves cardiaques artificielles, électrodes de stimulateur cardiaque, implants subcutanés et/ou implants intramusculaires et similaires.

20. Utilisation selon l'une des revendications 18 ou 19, **caractérisée en ce que** le matériau de revêtement est une solution, suspension ou émulsion d'une ou plusieurs substances actives ou états préalables de substance active dans un milieu support approprié.

21. Utilisation selon la revendication 20, **caractérisée en ce que** les substances actives ou état préalable de substances actives sont choisis parmi des substances pharmacologiquement actives, micro-organismes, matière cellulaire organique vivante, ainsi que des substances anorganiques ou organiques compatibles biologiquement.

22. Implant revêtu, pouvant être fabriqué suivant le procédé selon l'une des revendications 1 à 16.
